# EUROPEAN PATENT APPLICATION

(11) **EP 1 162 262 A1**
(43) Date of publication of application: **12.12.2001**
(21) Application number: 00906732.3
(22) Date of filing: 06.03.2000
(51) Int. Cl.: C12N 15/09, C12Q 1/68, G01N 33/50

(54) **FIBERS HAVING NUCLEIC ACID IMMOBILIZED THEREON, ALIGNMENT OF FIBERS HAVING NUCLEIC ACID IMMOBILIZED THEREON AND SLICE THEREOF**

(30) Priority: 05.03.1999 JP 5939799
(71) Applicant: Mitsubishi Rayon Co., Ltd., Tokyo 108-8506 (JP); Genox Research, Inc., Tsukuba Research Laboratories, Eisai Co., Ltd., Tsukuba-shi, Ibaraki 300-2635 (JP)
(72) Inventor: GUNJI, Shigemichi, Ota-ku, Tokyo 143-0024 (JP); YU, Fujio, Chemicals Development Lab., Mitsubishi, Yokohama-shi, Kanagawa 230-0053 (JP); AKITA, Takashi, Corporate Research Laboratories,, Otake-shi, Hiroshima 739-0693 (JP)
(74) Representative: HOFFMANN - EITLE
(86) International application number: JP0001352
(87) International publication number: WO0053739

(57) **Abstract**

By the present invention, there is provided a fiber having nucleic acid immobilized thereon, an alignment of fibers having nucleic acid immobilized thereon, and a slice thereof.

## Description

### Technical Field

The present invention relates to a high molecular material having nucleic acid immobilized thereon. Specifically, the present invention relates to fibers having nucleic acid immobilized thereon, an alignment of fibers having nucleic acid immobilized thereon, and a slice thereof.

### Background Art

Recently, genome projects have progressed in respect of various organisms and a large number of genes including human genes, as well as their nucleotide sequences, are rapidly being clarified. The functions of the genes for which sequences have been clarified are being examined with various methods, and as one of these methods, gene expression analysis employing clarified sequence information is known. For example, various methods have been developed, such as Northern hybridization, which employ nucleic acid - nucleic acid hybridization reactions or which employ PCR reaction. These various methods have enabled examination of the relationship between various genes and the organic function expression thereof. However, there is a limit to the number of genes to which these methods can be applied. Therefore, given a complex reaction system constituted by a very large number of genes such as those clarified at an individual level by genome projects, there are difficulties in performing a generalized and systematic gene analysis with the above methods.

Recently, a new analysis method and methodology known as the DNA microarray method (DNA chip method) which allows one-operation expression analysis of numerous genes, has been developed and now attracts attention.

This method does not differ in principle from conventional methods in respect of the fact that it is nucleic acid detection and assay method based on nucleic acid-nucleic acid hybridization. However, a major characteristic of this method is the utilization of a large number of DNA fragments aligned and immobilized at high density on a flat substrate slice called a microarray or chip. Examples of a specific method of using a microarray method include for example hybridizing a sample of expression genes of a test subject cell labeled with fluorescent pigment on a flat substrate slice, allowing mutually complimentary nucleic acids (DNA or RNA) to bind with one another and after labeling these locations with fluorescent pigment, rapidly reading with a high resolution analysis device. In this way, respective gene amounts in a sample can be rapidly estimated. That is, the essence of this new method is understood to be basically a combination of reduction of reaction sample amount and technology to arrange and align these reaction samples into a pattern allowing high volume, rapid, systematic analysis and quantification with good reproducibility.

Regarding techniques for immobilizing a nucleic acid on a substrate, apart from a method of high density immobilization on nylon sheets etc. such as in the above-mentioned Northern method, in order to further increase density, a method where polylysine is coated on a substrate of glass or the like, or a method involving direct solid phase synthesis of short-chain nucleic acids on a substrate of silicon or the like, are being developed.

However, while a spotting method of immobilization of nucleic acid on a substrate of glass or the like having an immobilization surface that is chemically or physically modified (*Science* 270, 467-470 (1995)) is superior to a sheet method in terms of spot density, it has been pointed out that in comparison to a direct synthesis method (U.S. Patent 5,445,934, U.S. Patent 5,774,305), spot density and amount of immobilized nucleic acid per spot are low and the method is inferior in terms of reproducibility. Alternatively, while a method involving solid phase synthesis of multiple short chain nucleic acids onto a silicon substrate in a regular manner using photolithography is superior in the number of types of nucleic acid able to be synthesized per unit of area (spot density), the amount immobilized per spot (synthesized amount), and reproducibility, the types of nucleic acid able to be immobilized are limited to relatively short-chained nucleic acids that are controllable with lithography. Further, it is difficult to effect a substantial reduction in cost per chip with this method due to the use of expensive manufacturing devices and multiple manufacturing steps. Also known as a method for solid phase synthesis of nucleic acid on a miniature carrier and library conversion thereof, is a method employing miniature beads. It is thought that this method enables synthesis of long chain nucleic acids with more types and at lower cost than a chip method, and also allows immobilization of longer nucleic acids such as cDNA, etc. However, differing from a chip method, it is difficult to produce a product such that specific compounds are arranged with good reproducibility according to a specific alignment standard.

### Disclosure of the Invention

Under such circumstances, the establishment of a new systematic methodology applicable to low cost mass manufacturing, enabling immobilization of nucleic acid at a specific concentration irrespective of chain length and enabling alignment in measurable form, with high density and good reproducibility, is strongly sought for gene analysis, which is considered to be a field that will grow in importance in the future. This is the problem which the present invention seeks to solve.

Specifically, the problem sought to be solved by the present invention is to establish a method of producing an alignment, i.e. that is, a two dimensional (planar) alignment having nucleic acids immobilized thereon (referred to as a 2-dimensional alignment of immobilized nucleic acids), which in comparison to methods for producing alignments of nucleic acid involving micro-spotting or micro-injection on two-dimensional substrates such as nylon sheets and glass substrate, has a high amount of immobilized nucleic acid, allows high densification of nucleic acid types arranged per unit of area, and is suitable for application of mass production. A further problem which the present invention seeks to solve is establishment of a production method for a two dimensional alignment of immobilized nucleic acids able to be applied to long chain nucleic acids including cDNA having lower production cost than methods of producing a high-density oligonucleotide alignment by a combination of photolithography onto a silicon substrate and solid phase synthesis.

Here, the object of the present invention is to provide a fiber having nucleic acid immobilized thereon, an alignment of fibers having nucleic acid immobilized thereon, and a slice thereof.

The present inventors, as a result of focused and deliberate research to solve the above problem have rethought the conventional paradigm of performing the nucleic acid alignment process and the immobilization process on the same two-dimensional carrier, and have discovered that it is possible to produce a two dimensional high density alignment of immobilized nucleic acids by independently performing the process of nucleic immobilization on a fiber being a one-dimensional construction (a single fiber), and by introducing various fiber shaping techniques into these alignment processes, producing a fiber bundle being a three-dimensional structure, and performing a slicing process on the obtained bundle.

That is to say, the present invention is a fiber incorporating a nucleic acid wherein the nucleic acid is immobilized on and/or in the fiber.

Further, the present invention is a fiber alignment comprising a bundle of the above-described fibers. Examples of said alignment include one where the fibers within the alignment are regularly arranged and one where there are more than one hundred fibers per cross-sectional 1cm². Further, the types of nucleic acid immobilized on these fibers may be different in respect of some or all of the fibers.

Further, the present invention is a slice of said fiber alignment, which intersects the fiber axis of the alignment.

This specification incorporates in its entirety the content described in the specification and drawings of Japanese Patent Application No. 11-59397, filed in 1999, which is a basis of a priority claim of the present application.

The present invention will be explained below.

In the present invention, examples of a nucleic acid to be immobilized on a fiber include deoxyribo nucleic acid (DNA) and ribonucleic acid (RNA). The nucleic acid used in the present invention can be commercially available nucleic acid or nucleic acid obtainable from viable cells etc.

Preparation of DNA or RNA from viable cells can be performed by known methods, for example by the method of Blin et al (Blin et al., *Nucleic Acids Res.* 3: 2303 (1976)), etc. in respect of DNA, or the method of Favaloro et al. (Favaloro et al., *Methods Enzymol*. *65:* 718 (1980)), etc. in respect of RNA. As a nucleic acid to be immobilized linear or circular plasmid DNA or chromosomal DNA, DNA fragments obtained by cleaving these with restriction enzymes or chemical cleaving, DNA synthesized by enzymes in a test tube, or chemically synthesized oligonucleotides.

In the present invention, nucleic acid may directly be immobilized on a fiber, or a derivative resulting from chemical modification of nucleic acid, or where necessary denatured nucleic acid may also be immobilized.

Examples of known chemical modifications able to be adopted in the present invention include amination, biotination and digoxygenin conversion (*Current Protocols in Molecular Biology,* Ed.; Frederick M. Ausubel et al. (1990), *Protocols for Experiments without Isotopes* (1) DIG hybridization (Syujyunsha)] By way of example, introduction of an amino group to the nucleic acid will be explained below.

A position for binding between an aliphatic hydrocarbon chain having an amino group and a single strand nucleic acid is not particularly limited, and is not only at the 5-terminus and 3-terminus of the nucleic acid, but can be within the nucleic acid chain (e.g. at a phosphate diester bond site or nucleotide binding site). This single chain nucleic acid derivative can be prepared according to the methods described in Japanese Patent Examined Publication (Kokoku) No. 3-74239, U.S. Patent No. 4,667,025, and U.S. Patent No. 4,789,737, etc. Apart from these methods, preparation can be conducted by using commercially available amination agents (e.g. Aminolink II (Trademark); PE Biosystems Japan, Amino Modifiers (Trademark), Clontech) or according to well-known methods for introducing an aliphatic hydrocarbon chain having an amino group to a DNA 5'-terminal phosphate (*Nucleic Acids Res.,* 11(18), 6513- (1983)).

In the present invention, examples of a fiber on which a nucleic acid can be immobilized include chemical fibers such as synthetic fibers, semi-synthetic fibers, regenerated fibers and inorganic fibers, and natural fibers.

Representative examples of synthetic fibers include various types of polyamide fiber including Nylon 6, Nylon 66, aromatic polyamides, etc., various polyester fiber such as polyethylene terephthalate, polybutylene terephthalate, polylactate, polyglycolic acid etc., various types of acryl fiber such as polyacrylonitrile etc., various types of polyolephine fiber such as polyethylene and polypropylene, various types of polyvinyl alcohol fiber, various types of polyvinylidene chloride fiber, various types of polyvinyl chloride fiber, various types of polyurethane fiber, various types of phenol fiber, fluoride fibers consisting of polyvinylidene fluoride and polytetrafluoroethylene, and various types of polyalkylene paraoxy benzoate fiber, etc. Further, non-clothing fibers such as optical fibers having e.g. transparent non-crystalline polymers such as polymethylmethacrylate and polystyrene as main components, can be used.

Representative examples of semi-synthetic fibers include various types of cellulose derivative fibers having diacetate, triacetate, chitin, or chitosan, etc. as raw materials, and various types of protein fiber known as promix, and the like.

Representative examples of regenerated fibers include various types of cellulose regenerated fibers (rayon, cupra, polynosic, etc.) obtainable by the viscous method, cuprammonium method, or organic solvent method

Representative examples of inorganic fibers include glass fibers, carbon fibers and the like.

Representative examples of natural fibers include plant fibers such as cotton, linen, ramie and jute, animal fibers such as wool and silk, and mineral fibers such as asbestos.

There is no particular limitation on the form of fiber to be used in the present invention. Further, the fiber can be a monofilament or a multifilament. Further, the fiber may be a yarn spun from staples. Further, where a multifilament or a fiber of spun yarn is used, voids between the individual fibers can be used in the immobilization of nucleic acid.

The fiber to be used in the present invention can be used, as is, in an unprocessed form or where necessary can be a fiber having reactive groups introduced thereto. Further, the fiber may be one which as been subjected to plasma processing or radiation processing with gamma rays or electron beams.

When immobilizing nucleic acid on these fibers, it is possible to exploit various chemical and physical interactions between the fiber and the nucleic acid, i.e. chemical and physical interactions between functional groups of the fiber and components which constitute nucleotides of the nucleic acid.

For example, when immobilizing unmodified nucleic acid on a fiber, it is possible to immobilize using baking or ultraviolet radiation after allowing the nucleic acid to act on the fiber. Further, when immobilizing nucleic acid modified with an amino group onto a fiber, binding with a functional group of a fiber can be accomplished by using a cross-linker such as glutalaldehyde or 1-ethyl-3-(3-dimethylaminopropyl)carbodiimide (EDC) or the like.

A temperature of 5 to 95°C is preferable, and of 15 to 60°C is more preferable when allowing a sample containing nucleic acid to act on a fiber. Processing time is normally between 5 minutes and 24 hours, and is preferably more than 1 hour.

A fiber having a nucleic acid immobilized thereon obtained by the above method can be subjected to various treatments as is appropriate. For example, by heat treatment, alkali treatment, surfactant treatment, or the like, it is possible to denature the immobilized nucleic acid. Or, where nucleic acid is obtained from living material such as cells, bacteria or the like, unwanted cell components and the like can be removed thereby. Then, post-treatment fibers can be used as a material for detecting nucleic acid. These processes may be performed separately or carried out at the same time. Further, these processes may be performed where appropriate on the sample containing nucleic acid prior to immobilization on a fiber.

A fiber having a nucleic acid immobilized thereon prepared as described above, is a basic unit constituting the fiber alignment of the present invention. These nucleic acid-immobilized fibers can be made into a fiber alignment by bundling together and adhering. Here, the nucleic acid- immobilized fibers are regularly arranged, and adhered with a resin adhesive or the like, to obtain a nucleic-acid immobilized alignment wherein nucleic acid-immobilized fibers are arranged in an orderly and regular manner lengthwise and breadthwise. The form of the fiber alignment is not particularly limited but usually by arranging the fibers in a regular manner, a square or rectangle is formed.

The term "regularly" is used to mean that fibers are arranged in an orderly manner so that the number of fibers contained in a frame of a certain size is the same. For example, where a bundle of fibers having a diameter of 1mm is arranged so that a section becomes a square 10mm long and 10mm wide, the number of fibers contained along an edge of the square frame (1cm²) is set at 10 fibers, and these fibers are tied in a line to obtain sheets, and then these sheets are overlaid to make 10 layers, and as a result, a total of 100 fibers, 10 lengthwise and 10 wide, can be arranged. However, a method in which fibers are regularly arranged is not limited to the above-stated overlaying of sheets.

In this case, it is preferable to align fibers in a form such that a fiber having a specific nucleic acid is at a pre-determined position. However, it is not absolutely necessary to align fibers in this manner. The reason for this is that even if the position of a fiber having a specific nucleic acid immobilized thereon is unclear when a fiber alignment is formed, it is possible to confirm the position of the fiber having a specific nucleic acid immobilized thereon by, after slicing a section of the alignment, determining the alignment position of the nucleic acid on said section. Therefore, if the positions of a plurality of nucleic acids arranged in a slice are determined once by this operation, since all of the slices obtained form a single alignment have identical positioning, the positions of nucleic acids in all slices obtained from the same alignment is understood.

Further, in the present invention, the number of fibers to be bundled together is 100 or more, preferably from 1,000 to 10,000,000, and can be set appropriately according to purpose. However, it is preferable that fiber density in the alignment is regulated to be from 100 to 1,000,000 fibers per 1cm². Further, thinner fibers are preferred in order to align fibers so as to obtained a slice of a fiber alignment having a nucleic acid immobilized thereon at high density. In a preferable embodiment of the present invention, it is necessary for width of a fiber to be less than 1mm or less. In the case of a monofilament, commercially available fishing line has a thickness of 50-900 µ m. Further, recent spinning technology has enabled production of a 1dtex monofilament (in the case of polyethylene terephthalate, a diameter of 14 µm). Even thinner fibers (superthin fibers, ultrathin fibers) are able to be produced (diameter: 1 to 10 µm).

Where a monofilament having a diameter of 50 µm is used, since it is possible to align 200 fibers per 1cm, the number of fibers which can be arranged in a 1cm² square is 40,000. Therefore, in this case it is possible to immobilize a maximum of 40,000 different kinds of nucleic acids per 1cm². On the other hand, multifilament e.g. 83 dtex/36 filament and 82 dtex/45 filament can be used intact.

Nucleic acids immobilized on each fiber within each alignment can each be a different type of nucleic acid. Alternatively, it is possible to select an arbitrary number of nucleic acids of the same type, and to bundle together and appropriately align the selected fibers. That is, according to the present invention, it is possible to arbitrarily establish the types and order of alignment of immobilized nucleic acids according to purpose.

In the present invention, a slice having a section of the nucleic acid immobilized alignment can be obtained by slicing the above mentioned nucleic acid immobilized alignment in a direction intersecting with, and preferably perpendicular to the fiber axis. Examples of a method of slicing in this case include a method of cutting away a slice using a microtome. Thickness of a slice can be freely regulated but is typically from 1 to 5,000 µm, preferably 10 to 2,000 µm.

In the thus obtained slice having a section of the nucleic acid-immobilized alignment, there exist nucleic acids according to the number of fibers constituting the alignment. Regarding the number of nucleic acids per sectional area of the slice, it is possible to produce a slice on which there immobilized 100 or more, or 1000 or more nucleic acids per 1cm² of the sectional area of a slice, depending on the choice of the external diameter of the fiber used and methods used when preparing the alignment.

These slices, with immobilized nucleic acids as probes, can be used to detect nucleic acids having a specific nucleotide sequence present in the sample, by allowing reaction with a sample and performing hybridization. In the present invention, a "probe" refers to a nucleic acid having a nucleotide sequence which is complementary to a nucleotide sequence of a gene to be detected. In other words, it is possible to detect nucleic acid possessing a target nucleotide sequence present in a sample by allowing a slice having a section of the nucleic acid-immobilized alignment of the present invention, to react and hybridize with a sample to form a hybrid; and detecting this hybrid.

For detection of a hybrid, it is possible to use known means capable of specifically recognizing as hybrid. For example, a label such as a fluorescent substance, light-emitting substance, radioisotope etc., can be allowed to act on sample nucleic acid, and this label can be detected. The types of these labels or the methods for introduction thereof are not limited in any way, and various conventionally known methods can be used.

It is possible to use these slices having immobilized nucleic acid as probes to detect a nucleic acid within a sample having a specific nucleotide sequence by allowing reaction and hybridization with a sample. In the present invention, "probe" refers to, in a narrow sense, a nucleic acid having a nucleotide sequence which is complementary to a nucleotide sequence of a gene to be detected. In other words, it is possible to detect nucleic acid possessing a target nucleotide sequence present in a sample by allowing a slice having a section of the nucleic acid-immobilized alignment of the present invention, to react and hybridize with a sample to form a hybrid of the probe and a complementary nucleic acid present in the sample; and detecting this hybrid. Further, in the present invention, "probe" refers to, in a broad sense, a nucleic acid capable of specifically binding with a protein or low molecular compound etc, present within a sample. Therefore, methods of using these slices are not limited to detection of nucleic acid which forms a hybrid with an immobilized nucleic acid (probe), but include detection of various samples of protein and low molecular compounds and the like (e.g. components of organisms, etc.) which specifically bind with the immobilized nucleic acid.

For detection of nucleic acids which form hybrids with immobilized nucleic acids, and detection of various components of organisms which bind specifically with immobilized nucleic acids, various known methods can be used. For example, for nucleic acids, proteins or low molecular compounds in a sample, a label such as a fluorescent substance, light-emitting substance or radioisotope can be allowed to act thereon, and this label can be detected. There is no particular limitation on the type of label or method of introducing a label, and various conventionally known means can be employed.

### Brief Description of Drawings

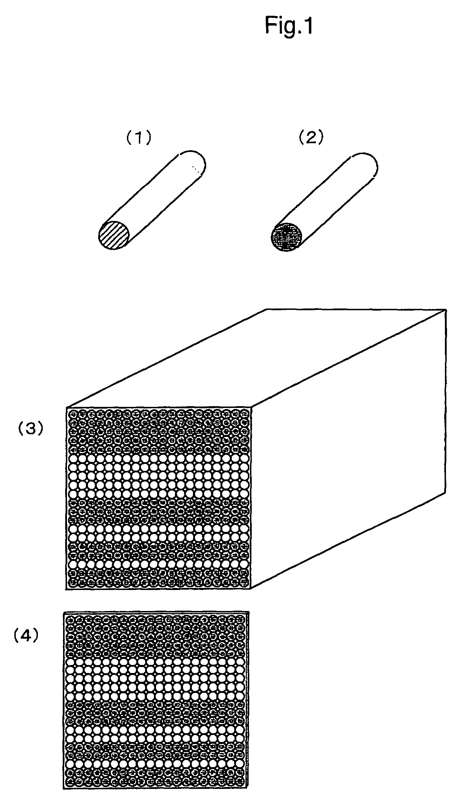

Figure 1 is a schematic diagram of a fiber having a nucleic acid immobilized thereon, an alignment of fibers having a nucleic acid immobilized thereon, and a slice thereof. In figure 1, (1) is a fiber having a nucleic acid immobilized thereon, on which Probe A is immobilized; (2) is a fiber having a nucleic acid immobilized thereon, on which Probe B is immobilized; (3) is alignment of fibers having a nucleic acid immobilized thereon consisting of these two types of fibers having a nucleic acid immobilized thereon; and (4) indicates a section of a nucleic-acid immobilized alignment sliced in a direction intersecting with the fiber axis of the alignment.

### BEST MODE FOR CARRYING OUT THE INVENTION

The present invention will be further explained below by use of Examples. However, the technical scope of the present invention is not to be limited by these Examples.

### Reference 1.

### Surface Treatment of a Fiber (1):

Nylon fiber (diameter 0.165mm, nylon 6 filament) was wound onto a polyethylene bobbin such that threads did not make contact with one another. This was immersed in formic acid (purity 99%) and held in the liquid for 10 seconds. Next, the nylon fiber wound on a yarn tube was pulled out and immediately immersed in water at room temperature. Thereafter, after thoroughly washing in running water, the fiber was dried to obtain a nylon fiber having a bleached surface. This nylon fiber was observed with a scanning electron microscope (JEOL, JSM-5300) by ordinary methods at an acceleration voltage of 5kV, and it was confirmed that the surface had been roughened.

### Reference 2

### Surface Treatment of a fiber (2):

By the same method as described in Reference 1, nylon fiber (diameter 0.165mm, nylon 6 filament) was immersed in 10% ethanol solution of sulfuric acid and treated to obtain a nylon fiber having a bleached surface. The surface of this nylon fiber was observed as in Reference 1 it was confirmed that the surface had been roughened.

### Reference 3

### Surface treatment of a fiber (3):

An acid anhydride group was introduced to a Nylon fiber (diameter: 0.165mm, Nylon 6 monofilament) according to the method described below.

An approximately 20mm nylon fiber was immersed in 10ml of 3mol/l HCl at 30°C for 30 minutes, and then washed with distilled water. After drying, the fiber was immersed in a 1:5 mixture of 10ml of 10%(w/v) polyethylene imine solution and methanol, for 30 minutes at room temperature. Thereafter, 20ml methanol solution containing 5% dicyclohexylcarbodiimide has added and the fiber was kept immersed for 2 hours. After washing with methanol and drying, the fiber was immersed for 2 hours at room temperature in a 20ml of dehydrated acetone solution containing 2%(w/v) anhydrous maleic acid-methylvinyl ether copolymer. After washing with acetone, the fiber was vacuum dried.

### Reference 4

Preparation of an oligonucleotide and an oligonucleotide having an amino group at the 5'-terminus thereof.

Oligonucleotides (Probe A, Probe B) were synthesized as described below.

Probe A: GCGATCGAAACCTTGCTGTACGAGCGAGGGCTC (SEQ ID NO:
1)
Probe B: GATGAGGTGGAGGTCAGGGTTTGGGACAGCAG (SEQ ID NO: 2)

Synthesis of oligonucleotides was performed using PE Biosystems' automatic synthesizer, DNA/RNA synthesizer (model 1394), and were used after deprotection and purification by standard techniques.

Further, aminated probes were also prepared by introducing NH₂(CH₂)₆- to the 5'-terminii of each oligonucleotide using Aminolink (Trademark) (Applied Biosystems) at the final step of DNA synthesis.

### Example 1

### Preparation of a fiber having a nucleic acid immobilized thereon (1):

The unmodified oligonucleotides prepared in Reference 4 were immobilized on each the nylon fibers subjected to surface processing in References 1 to 3 according to the following method.

The nylon fibers which were subjected to surface processing in Reference 1-3 were immersed in the oligonucleotide solution prepared in Reference 4 (nucleic acid concentration 10 µg/ml), and after drying in air, baking was performed at 80°C for 1 hour to obtain fibers having a nucleic acid immobilized thereon (figure 1). In figure 1, (1) is a fiber having Probe A immobilized thereon, (2) is a fiber having Probe B immobilized thereon, and in the fiber bundles of (3) and (4), fibers represented by white circles (○) are ones having probe A immobilized thereon, and those represent by black circles (●) are fibers having probe B immobilized thereon.

### Example 2 - Preparation of a fiber having a nucleic acid immobilized thereon (2):

The oligonucleotide having an amino group at the 5'-terminus thereof which was prepared in Reference 4 was immobilized on each the nylon fibers subjected to surface processing in References 1 to 3 according to the following method.

A 2500 µl solution of oligonucleotides having an amino group at the 5'-terminus thereof (nucleic acid concentration 10µg/ml, using phosphate buffer (pH8) containing 0.1M magnesium chloride as a solvent), 0.06g of 1-ethyl-3-(3-dimethylaminopropyl)-carbodiimide (EDC), and 500 µl of 1% weight nylon fiber were mixed and left to stand for 10 minutes. After washing with 50mM phosphate buffer (pH8.0) the fibers were immersed in 5ml of the same buffer. To this, 0.12g of EDC was added and after shaking for 3 hours at room temperature, the fibers were washed with 50mM phosphate buffer (pH8.0) thereby obtaining fibers having oligonucleotides immobilized thereon.

### Example 3

### Preparation of a fiber having a nucleic acid immobilized thereon (3):

The oligonucleotides having an amino group (Probe A and Probe B) which were prepared in Reference 4 were respectively immobilized on each on cellulose fibers (85decitex(dtex)/36 filament) according to the following method.

Cyanogen bromide (1g) was dissolved in 2ml of dimethyl formamide. This was added to an aqueous solution containing 5g of cellulose fiber having a length of 20cm, and allowed to stand at 15 to 20°C for 10 to 20 minutes. pH was maintained at 10.5-11.5 by adding 5mol/l sodium hydroxide. After reaction, the solution was washed with a volume of cooling water 15 times greater than that of the mixture, and finally washed with 10mM of phosphate buffer (pH8.0).

To a solution of 10mM phosphate buffer comprising cellulose fiber activated in this matter, oligonucleotides having an amino group as prepared in Reference4 (0.1 to 30mM) were added, and this was left to stand overnight at 20°C. After completion of the reaction, washing was performed with 10mM phosphate buffer (pH8.0), 1M phosphate buffer (pH8.0), 1M potassium chloride solution, and water, in that order. Thus a fiber having oligonucleotides immobilized thereon was obtained.

### Example 4:

### Preparation of an alignment of fibers having a nucleic acid immobilized thereon

Twenty of the nylon fibers having Probe A immobilized thereon obtained in Example 1 (ones having been subject to surface processing in Reference 1, length; 20cm) were aligned on a Teflon plate such that they adjoined one another but did not and both ends were fixed. To this, a thin coat of polyurethane resin adhesive (Nippon Polyurethane Industries Co.,Ltd., coronate4403, nippolan4223) was applied, and after the polyurethane resin had sufficiently solidified the fibers were removed from the Teflon plate, so as to obtain a sheet like product on which probe A-immobilized fibers were arranged in line. In the same manner, a sheet like product was also obtained for probe B-immobilized fibers. Then, twenty sheet-shaped product was laminated so as to form the arrangement of Figure 1(3). The above-mentioned adhesive was used to adhere, and a nucleic-acid immobilized fiber alignment was obtained having a total of 400 fibers, 20 fibers lengthwise and breadthwise respectively, arranged regularly and squarely.

Nucleic-acid immobilized fiber alignments were obtained also by similar operations in respect of the fibers subjected to surface processing in References 2 and 3, respectively.

Further, nucleic acid-immobilized fiber alignments were obtained also by similar operations in respect of the nucleic acid-immobilized fibers obtained in Examples 2 and 3, respectively.

### Example 5

### Preparation of a slice of an alignment of fibers having a nucleic acid immobilized thereon:

From the alignment of fibers having a nucleic acid immobilized thereon obtained in Example 4, a portion 100 µm thick was excised in a direction perpendicular to the fiber axis using a microtome, resulting in a slice of an alignment 20 x 20 fibers having a nucleic acid immobilized thereon wherein a total of 400 fibers, 20 fibers along each of the vertical and horizontal, are regularly and squarely aligned. (Figure 1(4)).

### Reference 5

### Labeling of sample nucleic acid:

As a model for sample nucleic acid, oligonucleotides (C, D) were synthesized which were complementary to a portion of the nucleotide sequences synthesized in Reference 4 (Probe A, Probe B).

Oligonucleotide C: GAGCCCTCGCTCGTACAGCAAGGTTTCG (SEQ ID NO.:
3)

Oligonucleotide D: CTGCTGTCCCAAACCCTGACCTCCACC (SEQ ID NO.:
4)

After introduction of NH₂(CH₂)₆- to the respective 5'-terminii of these oligonucleotides using Aminolink II (Trademark) (PE Biosystems Japan) as described in Reference 4, the 5'-terminii of these oligonucleotides were labeled with Digoxygenin (DIG, Roche Diagnostic Systems) according to the following procedure.

Terminal-aminated oligonucleotides were individually dissolved in 100mM borate buffer solution (pH 8.5) to a final concentration of 2mM. An equivalent of Digoxygenin-3-0-methylcarbonyl- ε-aminocapronic acid-N-hydroxy-succinimide ester (26mg/ml dimethyl formamide solution) was added to each and the mixture was allowed to stand overnight at room temperature.

An amount of the resultant mixture was adjusted to 100 µl, to which was added 2 µl of glycogen (Roche Diagnostics), 10 µl of 3M sodium acetate (pH 5.2) and 300 µl of cold ethanol, and then subjected to centrifugation at 15,000 rpm for 15 minutes, and the precipitate was collected. Further, 500 µl of 70% ethanol was added to the precipitate, and as a result of further centrifugation at 15,000rpm for 5 minutes, a precipitate collected at the bottom of a tube. This precipitate was dried with air and dissolved in 100 µl of 10mM Tris-HCl (pH7.5), 1mM EDTA.

The thus obtained DIG-labeled oligonucleotides were used as a model for sample nucleic acid.

### Reference 6

### Hybridization:

The nucleic acid-immobilized slice obtained in Example 5 was placed in a hybridization bag, hybridization solution consisting of the following components was poured thereinto, and pre-hybridization was performed for 30 minutes, at 45°C.

DIG-labeled DNA obtained in Reference Example 5 was added, and hybridization was performed at 45°C for 15 hours.

### Composition of Hybridization Solution:

5 x SSC (0.75M sodium chloride, 0.075M sodium citrate, pH 7.0)
5% blocking agent (Roche Diagnostics)
0.1% sodium N-lauroyl sarcocinate)
0.02% SDS (sodium lauroyl sulfate)
50% formamide

### Reference Example 7

### Detection:

After completion of hybridization, a slice having nucleic acid immobilized thereon was transferred to a pre-heated 50ml solution of 0.1 x SSC, 0.1% SDS and washed while shaking 3 times, at 45°C, for 20 min each time.

DIG buffer 1 was added, and SDS was removed while shaking at room temperature. This was repeated again before DIG buffer 2 was added and shaken for 1 hour. After removing the buffer, there was added 10 ml of solution containing 1/10,000 volumes of an anti-DIG alkaline phosphatase labeled antibody solution to DIG buffer 2, which was then gently shaken for 30 min so as to cause an antigen-antibody reaction. Next, this reaction solution was washed by shaking in DIG buffer 1 containing 0.2 % Tween 20 for 15 min twice, and subsequently immersed in DIG buffer 3 for 3 min. After removing DIG buffer 3, 3 ml of DIG buffer containing AMPPD was added to equilibrate for 10 min.

After draining, the resultant solution was transferred into a new hybridization bag, which was then left at 37 °C for 1 hour and bound together with a X ray film by means of a binder for X ray film, which film was then sensitized.

As a result, it was found that in each of them, oligonucleotide C bound to the site where probe A was placed and oligonucleotide D to the site where probe B was placed.
DIG buffer 1: 0.1 M maleic acid, 0.15 M sodium chloride (pH7.5).
DIG buffer 2: a buffer to which a blocking reagent is added at a concentration of 0.5%.
DIG buffer 3: 0.1 M Tris-HCl (pH9.5), 0.1 M sodium chloride, and 0.05 M magnesium chloride.
Blocking reagent: an anti-DIG alkaline phosphatase labeled antibody solution, AMPPD being a reagent included in DIG Detection kit (Roche Diagnostic Systems).

All publications, patents and patent applications cited within the present specification are incorporated herein by reference in their entirety.

### INDUSTRIAL APPLICABILITY

According to the present invention, there is provided a fiber having a nucleic acid immobilized thereon, an alignment of fibers having a nucleic acid immobilized thereon, and a slice thereof.

By the present invention, it is possible to obtain, efficiently and with good reproducibility, a slice have a fiber section of an alignment of fibers having a nucleic acid immobilized thereon, wherein nucleic acids are aligned precisely and at high density. Using this slice, it is possible to examine the type and amount of a nucleic acid within a sample.

When compared with conventional methods, the present invention has the advantages that by performing an immobilization process in an isolated and independent manner on a fiber as a one-dimensional structure rather than on a two-dimensional surface, it enables quantitative immobilization of nucleic acid independent of chain length. Further, the present invention allows higher densities to be achieved as a result of the introduction of fiber shaping and woven materials production techniques. Further, in order to prepare a target two-dimensional alignment from the resulting fiber bundle being a three-dimensional structure, a slicing process was newly introduced which did not exist in the prior art, thereby eliminating the need for micro-injection operations such as a spotting process that were error-prone, and enabling mass-production through continuous slicing.

### Sequence Listing Free Text:

SEQUENCE LISTING

<110> MITSUBISHI RAYON CO., LTD.
      GENOX RESERCH, Inc.

<120> NUCLEIC ACID-FIXED FIBER, AN ARRAY OF THE FIBERS AND A
       SLICE OF THE ARRAY.

<130> PH-788-PCT

<140> PCT/JP00/01352
<141> 2000-03-06

<150> JP11-59397
<151> 1999-03-05

<160> 4

<170> PatentIn Ver. 2.0

<210> 1
<211> 33
<212> DNA
<213> Artificial Sequence

<220>
<223> Synthetic DNA

<400> 1

gcgatcgaaa ccttgctgta cgagcgaggg ctc 33

<210> 2
<211> 32
<212> DNA
<213> Artificial Sequence

<220>
<223> Synthetic DNA

<400> 2
gatgaggtgg aggtcagggt ttgggacagc ag 32

<210> 3
<211> 28
<212> DNA
<213> Artificial Sequence

<220>
<223> Synthetic DNA

<400> 3
gagccctcgc tcgtacagca aggtttcg 28

<210> 4
<211> 27
<212> DNA
<213> Artificial Sequence

<220>
<223> Synthetic DNA

<400> 4
ctgctgtccc aaaccctgac ctccacc 27

SEQ ID NO.: 1 : Synthetic DNA
SEQ ID NO.: 2 : Synthetic DNA
SEQ ID NO.: 3 : Synthetic DNA
SEQ ID NO.: 4 : Synthetic DNA

## Claims

1. A fiber incorporating a nucleic acid, wherein the nucleic acid is immobilized on and/or in the fiber.

2. A fiber alignment comprising a bundle of the fibers according to claim 1.

3. The alignment according to claim 2, wherein the fibers within the alignment are regularly arranged.

4. The alignment according to claim 2 or 3, wherein the bundle of fibers comprises 100 or more fibers per cross-sectional 1cm².

5. The alignment according to any one of claims 2 to 4, wherein the type of nucleic acid is different in respect of some or all of the fibers.

6. A slice of fiber alignment of any one of claims 2 to 5, which intersects the fiber axis of the alignment.
